# EUROPEAN PATENT APPLICATION

(11) **EP 4 252 643 A1**
(43) Date of publication of application: **04.10.2023**
(21) Application number: 22165071.6
(22) Date of filing: 29.03.2022
(51) Int. Cl.: A61B 5/11, A61B 5/16, G10L 25/63, G06V 40/16, A61B 5/024

(54) **A SYSTEM AND METHOD FOR INTERPRETATION OF HUMAN INTERPERSONAL INTERACTION**

(71) Applicant: Emotion Comparator Systems Sweden AB, 106 91 Stockholm (SE)
(72) Inventor: HÖGMAN, Lennart, 113 40 Stockholm (SE)
(74) Representative: Zacco Sweden AB

(57) **Abstract**

The present disclosure relates to a system (100) and method for interpreting human interpersonal interaction. The system comprises first and second audio-visual stream generating devices (101a and 101b) each arranged to capture an audio-visual stream relating to at least one person during a session or a series of sessions, wherein the first and second audio-visual stream generating devices are synchronized, and a processor (102) arranged to process each audio-visual stream to identify non-verbal cues, such as facial, head and body movements, pupil size changes, and tone of voice, and map identified non-verbal cues in the first one of the audio-visual streams to corresponding, reactive, non-verbal cue in the second audio-visual stream and map identified non-verbal cues in the second one of the audio-visual streams to corresponding, reactive, non-verbal cue in the first audio-visual stream to thereby identify a non-verbal communication pattern.

## Description

### TECHNICAL FIELD

The present disclosure relates to a system and method for human interaction by using first and second audio-visual stream generating devices (101a 101b), wherein each audio-visual stream generating device is arranged to capture an audio-visual stream relating to at least one person during a session.

### BACKGROUND

Analysis of interpersonal communication is extremely complex. In settings like psychotherapy, negotiations, interviews etc. interpersonal communication is crucial for reaching goals.

There are today technologies to capture and analyse both verbal and body language at a detailed level. The focus has mainly been on Natural Language Processing, NLP, and Facial Expression Recognition.

WO 2017/216758 relates to a computer-implemented method of digital image analysis. The method includes obtaining first digital video of a human subject that indicates facial expressions of the human subject; performing micro-expression analysis on the human subject using the first digital video; comparing results of the performed micro-expression analysis with content of a presentation determined to have been provided to the human subject at the same time that particular portions of the digital video were initially captured; and modifying a manner of performing interaction with the human subject or other human subjects based on the comparing of results.

### SUMMARY

The present disclosure relates to analysis of interpersonal communication that will be applied in physical as well as digital meetings at a level of detail that has not previously been achieved. In settings like psychotherapy, negotiations, interviews etc. interpersonal communication is crucial for reaching goals. As yet, analysis of communication is extremely complex, being multimodal, including conscious as well as unconscious communicative elements that take place on different time scales. Those communicative elements may comprise temporal shifts as for instance turn taking, mimicry patterns, shifting roles from actor to reactor, signs of dominance, benevolence, trust, distrust, etc. These communicative elements may be manifest or subtle as for instance including non-conscious micro-expressions. Even for a trained person like a psychotherapist it is not possible to pick up all information while communication is ongoing, this is also true for post session video analyses.

There are today technologies to capture and analyse both verbal and body language at a detailed level, but these have not been systematically integrated and used to their full capacity.

So far, technological interpretation of interpersonal communication has generally not utilized multimodal analytical techniques. In accordance with the present disclosure, all available non-verbal and verbal communication cues, can be utilized.

An object of the present invention is to alleviate at least some of the problems as described above.

This has been achieved by means of a system for interpreting human interpersonal interaction. The system comprises first and second audio-visual stream generating devices each arranged to capture an audio-visual stream relating to at least one person during a session or a series of sessions, wherein the first and second audio-visual stream generating devices are synchronized. The system further comprises a processor arranged to process each audio-visual stream to identify non-verbal cues in the respective audio visual stream. The processor is further arranged to map identified non-verbal cues in the first one of the audio-visual streams to corresponding reactive non-verbal cue in the second audio-visual stream and to map identified non-verbal cues in the second one of the audio-visual streams to corresponding, reactive non-verbal cue in the first audio-visual stream to thereby identify a non-verbal communication pattern.

The non-verbal cues comprise for example facial, head and body movements, pupil size changes, and tone of voice.

The non-verbal cues may have one of the flowing relations to a verbal message.
- It repeats and strengthens the verbal message
- It contradicts the verbal message
- It substitutes the verbal message
- It complements and accents the verbal message. For example, facial expressions often conveys a far more vivid message than words.

The mapping of identified non-verbal cues in the first one of the audio-visual streams to corresponding, reactive non-verbal cue in the second audio-visual stream and the mapping of identified non-verbal cues in the second one of the audio-visual streams to corresponding, reactive non-verbal cue in the first audio-visual stream may comprise analyzing how much of variance that could be explained in non-verbal cues from person A to B and vice versa and based thereon determine whether a non-verbal cue is a reactive non-verbal cue or a non-reactive non-verbal cue. In this regard, different time windows and time lags may be used.

In this context, a time lag is characteristically a time delay used for identifying mirroring. The time lag is characteristically longer than 200 ms. Due to the fact that emotional expressions may be activated by different brain networks with different processing speeds, the time lag for a spontaneous mirroring action is smaller than for acted or social mirroring. For example, a spontaneous mirroring of a smile is characteristically about 200 - 400 ms faster than an acted or social smile.

The term time window represents a time window, sequence or time segment during which the analysis takes place. For example, the time window may be selected as 5-10 seconds. Further, or instead, the selection of the time window may depend on what is going on in the interaction between the persons. The time window can be selected manually or determined by an algorithm, such as an algorithm based on Machine Learning.

In one option, the processor is arranged to monitor a plurality of predefined action units in the first and second audio-visual stream, the respective action unit corresponding to a part of the face or a part of the body or a characteristic in the voice. The processor is then arranged to identify the non - verbal cues based on characteristics identified in the respective predefined action unit.

The mapping of identified non-verbal cues in the first one of the audio-visual streams to corresponding, reactive non-verbal cue in the second audio-visual stream and the mapping of identified non-verbal cues in the second one of the audio-visual streams to corresponding, reactive non-verbal cue in the first audio-visual stream may comprise doing the mapping for each predefined action unit and then determine a degree of dependence between different action units. The determination of a degree of dependence between different action units comprises determining how much the pattern of non-verbal cues for the first and second audio-visual streams for one action unit correlates to the corresponding pattern for other action units.

This may as discussed above comprise analyzing how much of variance that could be explained in non-verbal cues from person A to B and vice versa and based thereon determine whether a non-verbal cue is a reactive non-verbal cue or a non-reactive non-verbal cue. In this regard, different time windows and time lags may be used.

For example, time series analysis first of all pairs of non-verbal cues on corresponding action units may be made (for instance facial action unit AU 23 from A to B and vice versa) and then time series analysis may be made on all possible combinations of action units to determine presence of reactive non-verbal cues in any action unit or in preselected action units. Thus, this can be explained as identified non-verbal cues in each action unit of the n action units in one of the audio-visual streams is mapped to reactive non-verbal cues in the n action units in the other audio-visual stream.

For example, in order to determine reactive non-verbal cues, algorithms may be used based on granger causality. Granger causality is a statistical concept of causality that is based on prediction. According to Granger causality, if a signal X1 "Granger-causes" (or "G-causes") a signal X2, then past values of X1 should contain information that helps predict X2 above and beyond the information contained in past values of X2 alone. Its mathematical formulation is based on linear regression modeling of stochastic processes (Granger 1969). For example, bidirectional long short-term memory Granger causality (bi-LSTM-GC) calculations may be used. The algorithms used for determining reactive non-verbal cues may be implemented with a recurrent neural network, RNN.

The action units may comprise at least one facial action unit corresponding to a predetermined part of the face, wherein for the facial action unit is defined for a set of coordinates or a relation between coordinates of the predetermined part of the face, wherein the non - verbal cues are determined based on a temporary change in the set of coordinates or a temporary change in relation between coordinates. The coordinate system is head centred.

The action units, at least for facial action units, may for example be defined using the Facial Action Coding System, FACS. To sum up, in accordance with FACS, movements of individual facial muscles are encoded by FACS from slight different instant changes in facial appearance. FACS is used to systematically categorize the physical expression of emotions. The system has proven useful to psychologists and to animators. FACS is a computed automated system that detects faces in videos, extracts the geometrical features of the faces, and then produces temporal profiles of each facial movement.

Accordingly, a movement in the set of coordinates or a change in relation between coordinates of the predetermined part of the face is determined for the respective facial action unit. Other facial action units may relate to facial movements such as eye movements and also blinking rate and changes of pupil size.

The action units may comprise at least one body action unit corresponding to a predetermined part of the body, wherein the body action unit comprises a set of coordinates or a relation between coordinates of the predetermined part of the body, wherein the non - verbal cues are determined based on a temporary change in the set of coordinates or relation between coordinates.

The body action units may relate to body movements and body posture. The non-verbal cues which can be determined may for example reflect approach avoidance, signs of arousal, etc. The body action units may also comprise heart rate and heat rate variability, HRV, other psychophysiological data and prosody.

The voice action units may comprise at least one voice characteristic such as
- a rate of loudness peaks, i.e., the number of loudness peaks per second,
- a mean length and standard deviation of continuously voiced regions,
- a mean length and standard deviation of unvoiced regions,
- the number of continuous voiced regions per second,
   wherein the non - verbal cues are determined based on a temporary change the voice characteristics.

As is understood from the above, in an option, the processor is arranged to, for each action unit compare the evolution of the first and second audio-visual streams with regards to activation of non-verbal cues, to determine a time lag between activations of non-verbal cues in the first and second audio-visual streams and to, based on the determined time lags, determine occasions of activations and non-activations of reactive non-verbal cues.

The processor may be arranged to, based on the determined time lags between activations of non-verbal cues in the first and second audio-visual streams for one or a plurality of action units, determine whether the reactive cues are spontaneous or consciously controlled.

The processor may be arranged to, based on determined occasions of activations and non-activations of reactive non-verbal cues for one or a plurality of action units, determine whether there is a dynamic in the interaction and/or determined whether any of the persons has a dynamic behaviour.

In an option, the processor is arranged to analyse the identified communication pattern to categorize psycho-social states of the respective person, said psycho-social states comprising at least one of emotion, attention pro-social, dominance and mirroring, said analyses being performed in a rolling window time series, wherein the time series is from 0.1 s and more, for example in the interval 0.2-10s.

The categorisation of states can be made based on heuristics as well as on Machine Learning, ML, models. At least one of the following heuristics models based on earlier research may be used These are examples and other heuristics may be used.
- Positive mirroring within a time-window of 200-400 ms may be a spontaneous pro-social signal. A spontaneous mirroring is characteristically at least 200-300 ms faster than deliberate or social mirroring.
- Positive mirroring within a time-window of 500 -2000 ms may be determined as deliberate or social mirroring expression
- Spontaneous mirroring of a smile is characteristically about 200 - 400 ms faster than an acted or social smile
- A smile activated at the left side before the right side indicates that the smile is genuine,
- Lack of mirroring of positive signals may be a sign of distrust or lack of emotional reactivity
- Leaning forward while showing a spontaneous positive expression is a pro-social signal
- Leaning forward while showing a spontaneous negative expression is an aggressive signal.

At least some of the models above can be combined with trained ML models based on annotated (ground truth) data from psychotherapy sessions.

The system may further comprise a presentation device arranged to present information relating to the non-verbal communication pattern, such as the categorized psycho-social states of the respective person during the session.

### DESCRIPTION OF DRAWING

Figure 1 is a block scheme showing an example system system for interpreting human interaction
Figure 2 illustrates an example set-up for a session using a system as disclosed in figure 1.
Figure 3 illustrates examples of facial action units.
Figure 4 illustrates examples of body action units.
Figure 5 illustrates an example for finding reactive non-verbal cues.
Figure 6 illustrates an example method for interpretation of human interaction.

### DETAILED DESCRIPTION

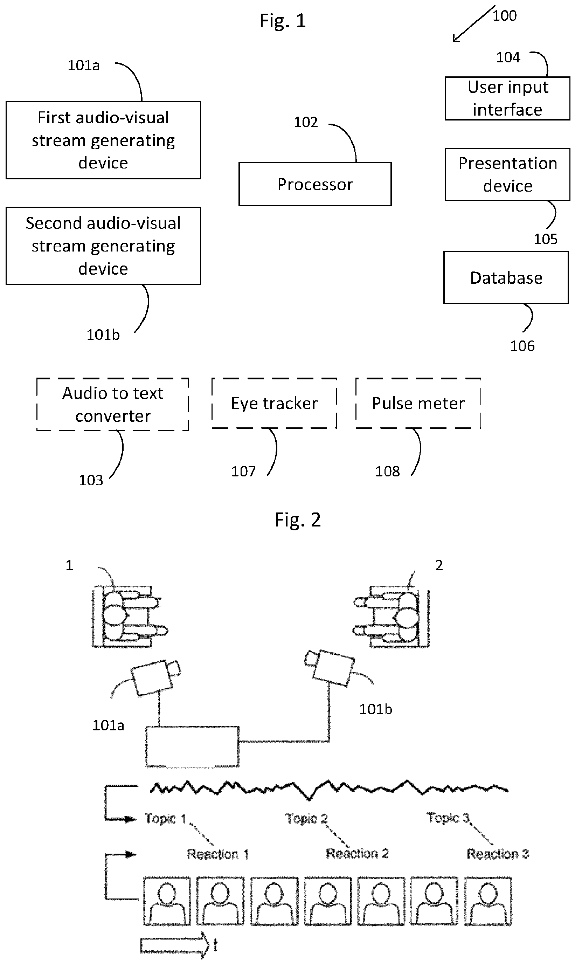

In figure 1 a system 100 is disclosed for interpreting human interpersonal interaction. The system 100 comprises first and second audio-visual stream generating devices 101a and 101b, each arranged to capture an audio-visual stream relating to at least one person during a session or a series of sessions. The sessions may be sessions where the participants participate in the same room or via an online meeting. In the online meeting, the participants may participate via a computer for example using Microsoft Teams or Zoom. When the session is an online digital meeting it is pre-assumed that the participants are able to both see each other and hear each other via the computer. The first and second audio-visual streams as discussed above may in this context be either the audio-visual stream(s) of the online meeting. Alternatively, a separate device for generating the herein discussed audio-visual stream arranged at the respective participant's facility may be used.

The first and second audio-visual stream generating devices are synchronized.

The system 100 comprises further at least one processor 102 arranged to process each audio-visual stream to identify non-verbal cues, such as facial, head and body movements, pupil size changes, and tone of voice, and map identified non-verbal cues in the first one of the audio-visual streams to corresponding, reactive, non-verbal cue in the second audio-visual stream and map identified non-verbal cues in the second one of the audio-visual streams to corresponding, reactive, non-verbal cue in the first audio-visual stream to thereby identify a non-verbal communication pattern.

The processor 102 may comprise an AI algorithm arranged to identify the non-verbal communication pattern and/or information relating to the non-verbal communication pattern.

The system may further comprise a presentation device 105 arranged to present to a user information relating to the non-verbal communication pattern. The presentation device may be arranged to present a first set of information relating to the non-verbal communication pattern during the session. This first set of information is generally information which can be calculated and presented in near real time. The presentation device may be arranged to present a second set of information relating to the non-verbal communication pattern based on an in-depth analysis after a session. In an example, a first, preferably local, processor is arranged to calculate the first set of information. Further, a second, local, remote or cloud based processor may be arranged to calculate the second set of information. The presentation device may include a computer screen associated to the processor. The presentation device may be any type of display arranged to present the first and/or second set of information. For example, the presentation device may be connected to a web-based interface and for example implemented as an app.

The information relating to the non-verbal communication pattern may for example include categorized psycho-social states of at least one of the persons during the session.

For example, the information relating to the non-verbal communication pattern may comprise:
- all emotions actually displayed by at least one of the persons
- mixture of different emotions displayed by at least one of the persons in real time
- non-dominant emotions that are ongoing and are displayed by at least one of the persons in addition to dominant emotion.

At least one of the above examples of information relating to the non-verbal communication pattern may be included in the first set of information. Typically displayed emotions include intensity, happiness, sadness, anger, surprise, fear disgust and combinations, such as happily surprised. Also mirroring may be displayed by the persons - e.g. when the interactants are showing the same expression.

The one or more of the following information may be included in the second set of information.
- all emotions,
- micro expressions,
- eye movements,
- body movements,
- heart rate and other psychophysiological data,
- prosody, and
- verbal content.

The above information relate to non-displayed information, i.e. information which is not directly derivable from the audio-visual streams. Instead, a plurality of simultaneous processes may be identified in the minds of the interactants characteristically having completely different temporal dynamics. It is a challenge to weighting these processes together into a whole that has an explanatory value. Machine learning is preferably used in order to make this processing.

In an example, the system further comprises a converter 103 arranged to convert verbal content in at least one of the audio-visual streams to text. Thereby, a transcript of the conversation can be provided.

The at least one processor may be arranged to add or associate at least some of the first and/or second set of information as discussed above to the corresponding part of the transcript of the text. Thereby an enriched transcript is provided where all or some of the non-verbal information is added to the text.

The presentation device 105 may then be arranged to present such enriched transcript of the text.

The at least one processor may further be arranged to compare the text and non-verbal information to identify any incoherent non-verbal signals. The at least one processor may further be arranged to analyse the non-verbal information only to identify any incoherence between non-verbal signals.

The presentation device 105 may then be arranged to present any such identified incoherences. The presentation of the incoherences may be made in the enriched transcript of the text or in any other way.

Further, the text and identified non-verbal information may even be analysed to predict an outcome or to identify critical events in a session. The presentation device 105 may then be arranged to present any such outcome and/or identified critical event. The presentation of the outcome and/or identified critical event may be made in the enriched transcript of the text or in any other way.

The system may further comprise a user input interface 104 for user input of at least one of
- session data such as background data, session type, and script/scheme for session,
- data associated to at least one of the audio-visual streams, such as timestamps and short notes,
- marks in the transcript of the text of emotionally important passages, and
- post session data such as interpersonal ratings and task performances.

In the illustrated example of figure 1, the system comprises an eye tracker 107.

In the illustrated example of figure 1, the system comprises a pulse meter 108.

In the illustrated example of figure 1, the system further comprises a database 106. The database may be used to store at least some of the information/data provided using the system as described herein.

In figure 2, an example set-up for a session is illustrated with a first person 1 and a second person 2 participating.

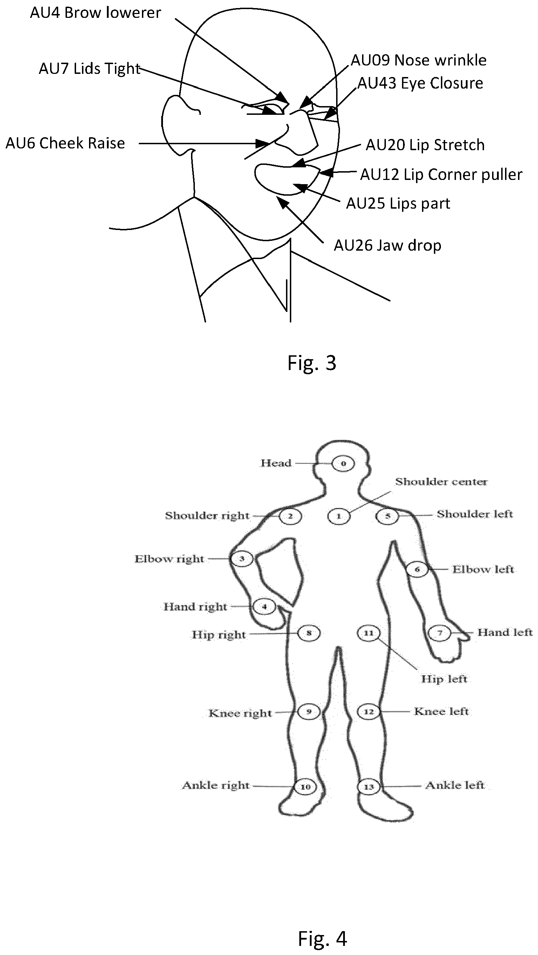

In figures 3 and 4, examples for identifications of non-verbal cues are illustrated. The processor of the system as disclosed in relation to figure 1 is arranged to monitor a plurality of predefined action units in the first and second audio-visual stream. The respective action unit corresponding to a part of the face or a part of the body or a characteristic in the voice. The processor is arranged to identify the non - verbal cues based on characteristics identified in the respective predefined action unit.

The processor may be arranged to, for each action unit, compare the evolution of the first and second audio-visual streams with regards to activation of non-verbal cues, and to determine a time lag between activations of non-verbal cues in the first and second audio-visual streams and to, based on the determined time lags, determine occasions of activations and non-activations of reactive non-verbal cues.

The processor may be arranged to, based on the determined time lags between activations of non-verbal cues in the first and second audio-visual streams for one or a plurality of action units, determine whether the reactive cues are spontaneous or consciously controlled.

The processor may be arranged to, based on determined occasions of activations and non-activations of reactive non-verbal cues for one or a plurality of action units, determine whether there is a dynamic in the interaction and/or determined whether any of the persons has a dynamic behaviour.

The respective action units may in a simple example have an activated and non-activated state. However, preferably, the state of the action units is defined by its coordinate(s) or relative coordinate(s). Thus, the number of states of the respective action unit is not limited.

In the examples illustrated in figure 3, the action units comprise at least one facial action unit corresponding to a predetermined part of the face. The facial action unit comprises a set of coordinates or a relation between coordinates of the predetermined part of the face, wherein the non - verbal cues are determined based on a temporary change in the set of coordinates or relation between coordinates.

In the illustrated example the following facial action units are illustrated
AU4 Brow Lowerer
AU6 Cheek raise
AU7 Lids Tight
AU9 Nose Wrinkle
AU12 Lip Corner Puller
AU25 Lips Part
AU26 Jaw Drop
AU43 Eye closure

It is then for example determined for each facial action unit how the coordinates move in relation to a head centred coordinate system.

Thus, the action units may comprise at least one facial action unit corresponding to a predetermined part of the face, wherein for the facial action unit is defined for a set of coordinates or a relation between coordinates of the predetermined part of the face, wherein the non - verbal cues are determined based on a temporary change in the set of coordinates or a temporary change in relation between coordinates. The coordinate system is head centred.

The action units, at least for facial action units, may for example be defined using the Facial Action Coding System, FACS. To sum up, in accordance with FACS, movements of individual facial muscles are encoded by FACS from slight different instant changes in facial appearance. FACS is used to systematically categorize the physical expression of emotions. The system has proven useful to psychologists and to animators. FACS is a computed automated system that detects faces in videos, extracts the geometrical features of the faces, and then produces temporal profiles of each facial movement.

Accordingly, a movement in the set of coordinates or a change in relation between coordinates of the predetermined part of the face is determined for the respective facial action unit.

Note that facial action units may be provided at both the left and right side of the face. Non-verbal information may be provided from a relation between left side and right side action units. For example, a smile activated at the left side before being activated at the right side indicates that the smile is genuine.

Other facial action units may relate to facial movements such as eye movements and also blinking rate and changes of pupil size. Data related to those other facial action units may be provided from for example an eye tracker.

In the example, of figure 4, the action units comprise at least one body action unit corresponding to a predetermined part of the body. The respective body action unit comprises a set of coordinates or a relation between coordinates of the predetermined part of the body, wherein the non - verbal cues are determined based on a temporary change in the set of coordinates or relation between coordinates.

In the illustrated examples the following example body action units are illustrated:
- head
- shoulder center
- shoulder left
- shoulder right
- elbow left
- elbow right
- hand left
- hand right
- hip left
- hip rignt
- knee left
- knee right
- ankle left
- ankle right

Other body action units may be provided from for example a pulse meter.

In a non-illustrated example, wherein the action units comprises at least one voice characteristic such as
- a rate of loudness peaks, i.e., the number of loudness peaks per second,
- a mean length and standard deviation of continuously voiced regions,
- a mean length and standard deviation of unvoiced regions,
- the number of continuous voiced regions per second,
wherein the non - verbal cues are determined based on a temporary change the voice characteristics. This information is provided from the audio part of the respective audio-visual stream.

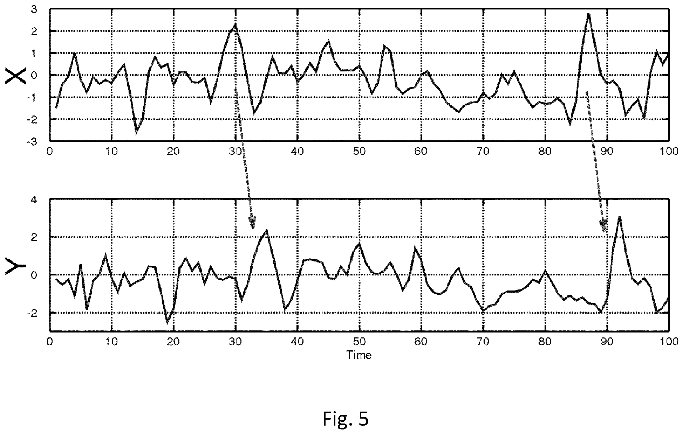

In figure 5, an example is illustrated for finding reactive non-verbal cues is illustrated. For each action unit the evolution of the first and second audio-visual streams are compared with regards to activation of non-verbal cues, to determine a time lag between activations of non-verbal cues in the first and second audio-visual streams and to, based on the determined time lags, determine occasions of activations and non-activations of reactive non-verbal cues.

In the detailed granger causality-based example, when time series X Granger-causes time series Y, the patterns in X are approximately repeated in Y after some time lag (two examples are indicated with arrows). Thus, past values of X can be used for the prediction of future values of Y Frequency related parameters:
- Pitch, logarithmic F0 on a semitone frequency scale, starting at 27.5 Hz (semitone 0).
- Jitter, deviations in individual consecutive F0 period lengths.
- Formant 1, 2, and 3 frequency, centre frequency of first, second, and third formant
- Formant 1, bandwidth of first formant. Energy/Amplitude related parameters:
- Shimmer, difference of the peak amplitudes of consecutive F0 periods. Loudness, estimate of perceived signal intensity from an auditory spectrum.
- Harmonics-to-noise ratio (HNR), relation of energy in harmonic components to energy in noise-like components. Spectral (balance) parameters: Alpha Ratio, ratio of the summed energy from 50-1000 Hz and 1-5 kHz
- Hammarberg Index, ratio of the strongest energy peak in the 0-2 kHz region to the strongest peak in the 2-5 kHz region.
- Spectral Slope 0-500 Hz and 500-1500 Hz, linear regression slope of the logarithmic power spectrum within the two given bands.
- Formant 1, 2, and 3 relative energy, as well as the ratio of the energy of the spectral harmonic peak at the first, second, third formant's centre frequency to the energy of the spectral peak at F0.

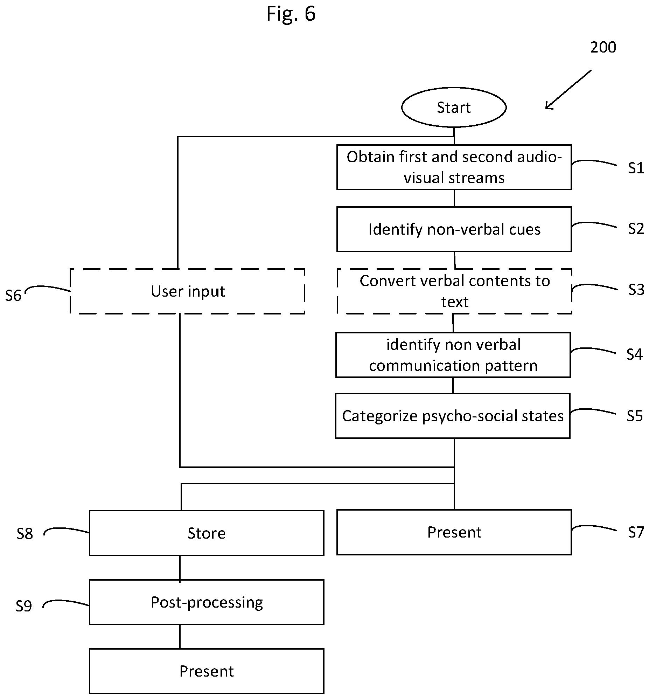

In figure 6, an example method is illustrated for interpreting human interpersonal interaction. The method is characteristically computer implemented. The method comprises:
- obtaining S1 synchronized first and second audio-visual streams, each audio-visual stream relating to at least one person during a session,
- processing S2 said first and second audio-visual streams to identify (S2) non-verbal cues, such as facial, head and body movements, pupil size changes, and tone of voice, and
- comparing the audio-visual streams to map identified non-verbal cues in one of the audio-visual streams with non-verbal cues in the other audio-visual stream, to thereby identify S4 an non-verbal communication pattern .

The method may further comprise a step of analysing the identified communication pattern to categorize (S5) psycho-social states of the respective person, said psycho-social states comprising at least one of emotion, attention pro-social, dominance and mirroring, said analyses being performed in a rolling window time series, wherein the time series is user set or set by an algorithm. The interval is for example 0.2-10s.

The method may further comprise a step of presenting (S7) information relating to the non-verbal communication pattern, such as the categorized psycho-social states of the respective person during the session.

The method may further comprise receiving S6, via a user input interface for user input, at least one of
- session data such as background data, session type, and script/scheme for session,
- data associated to at least one of the audio-visual streams, such as timestamps or other markers and/or notes and/or predefined tabs, and
- post session data such as interpersonal ratings and task performances.

The method may further comprise storing S8 in a database at least one of
a. the first and/or second audio-visual stream,
b. the identified non-verbal cues in the first and/or second audio-visual stream
c. the categorized psycho-social states,
d. at least a part of the contents of the first and/or second audio-visual stream converted to text,
e. received user input data
f. additional physiological data obtained from additional sensors
g. an enriched transcript of a text from the communication during the session, wherein all or some of the non-verbal information is added to the text.

The method may further comprise a step of post-processing (S9) data for a plurality of sessions in the database, said post-processing comprising at least one of
- identification of reaction patterns in a session which leads to a non-favourable result based on previous session series, and
- determine how mirroring patterns develop over time.

## Claims

1. A system (100) for interpreting human interpersonal interaction, comprising: first and second audio-visual stream generating devices (101a and 101b) each arranged to capture an audio-visual stream relating to at least one person during a session or a series of sessions,
wherein the first and second audio-visual stream generating devices are synchronized, and
a processor (102) arranged to
process each audio-visual stream to identify non-verbal cues, such as facial, head and body movements, pupil size changes, and tone of voice, and
map identified non-verbal cues in the first one of the audio-visual streams to corresponding, reactive, non-verbal cue in the second audio-visual stream and map identified non-verbal cues in the second one of the audio-visual streams to corresponding, reactive, non-verbal cue in the first audio-visual stream to thereby identify a non-verbal communication pattern.

2. The system according to claim 1, wherein the processor is arranged to monitor a plurality of predefined action units in the first and second audio-visual stream, the respective action unit corresponding to a part of the face or a part of the body or a characteristic in the voice and wherein the processor is arranged to identify the non - verbal cues based on characteristics identified in the respective predefined action unit.

3. The system according to claim 2, wherein the action units comprise at least one facial action unit corresponding to a predetermined part of the face, wherein the facial action unit comprises a set of coordinates or a relation between coordinates of the predetermined part of the face, wherein the non - verbal cues are determined based on a temporary change in the set of coordinates or relation between coordinates.

4. The system according to claim 2 or 3, wherein the action units comprises at least one body action unit corresponding to a predetermined part of the body, wherein the body action unit comprises a set of coordinates or a relation between coordinates of the predetermined part of the body, wherein the non - verbal cues are determined based on a temporary change in the set of coordinates or relation between coordinates.

5. The system according to any of the claims 2 to 4, wherein the action units comprises at least one voice characteristic such as
• a rate of loudness peaks, i.e., the number of loudness peaks per second,
• a mean length and standard deviation of continuously voiced regions,
• a mean length and standard deviation of unvoiced regions,
• the number of continuous voiced regions per second,
wherein the non - verbal cues are determined based on a temporary change the voice characteristics.

6. The system according to any of the claims 2 - 5, wherein the processor is arranged to, for each action unit compare the evolution of the first and second audio-visual streams with regards to activation of non-verbal cues, to determine a time lag between activations of non-verbal cues in the first and second audio-visual streams and to based on the determined time lags determine occasions of activations and non-activations of reactive non-verbal cues,
where the processor optionally is arranged to, based on the determined time lags between activations of non-verbal cues in the first and second audio-visual streams for one or a plurality of action units, determine whether the reactive cues are spontaneous or consciously controlled.

7. The system according to claim 6, wherein the processor is arranged to, based on determined occasions of activations and non-activations of reactive non-verbal cues for one or a plurality of action units, determine whether there is a dynamic in the interaction and/or determined whether any of the persons has a dynamic behaviour.

8. The system according to any of the preceding claims, wherein the processor (102) comprises an AI algorithm arranged to identify the non-verbal communication pattern.

9. The system according to any of the preceding claims, wherein the processor is arranged to analyse the identified non-verbal communication pattern to categorize psycho-social states of the respective person, said psycho-social states comprising at least one of emotion, attention pro-social, dominance and mirroring, said analyses being performed in a rolling window time series, wherein the time series is from 0.1 s and more, for example in the interval 0.2-10s.

10. The system according to any of the preceding claims, further comprising a presentation device (105) arranged to present to a user information relating to the non-verbal communication pattern, such as the categorized psycho-social states of at least one of the persons during the session.

11. A method (200) for interpreting human interaction, comprising:
obtaining (S1) synchronized first and second audio-visual streams, each audio-visual stream relating to at least one person during a session,
processing (S2) said first and second audio-visual streams to identify (S2) non-verbal cues, such as facial, head and body movements, pupil size changes, and tone of voice, and
comparing the audio-visual streams to map identified non-verbal cues in one of the audio-visual streams with non-verbal cues in the other audio-visual stream, to thereby identify (S4) an non-verbal communication pattern.

12. The method according to claim 11, further comprising a step of analysing the identified communication pattern to categorize (S5) psycho-social states of the respective person, said psycho-social states comprising at least one of emotion, attention pro-social, dominance and mirroring, said analyses being performed in a rolling window time series, wherein the time series optionally is in the interval 0.2-10s.

13. The method according to claim 11 or 12, further comprising a step of presenting (S7) information relating to the non-verbal communication pattern, such as the categorized psycho-social states of the respective person during the session.

14. The system according to any of the claims 11 - 13, further comprising receiving (S6), via a user input interface for user input, at least one of
session data such as background data, session type, and script/scheme for session,
data associated to at least one of the audio-visual streams, such as timestamps or other markers and/or notes and/or predefined tabs, and
post session data such as interpersonal ratings and task performances.

15. The method according to any of the claims 11-14, further comprising storing (S8) in a database at least one of
a. the first and/or second audio-visual stream,
b. the identified non-verbal cues in the first and/or second audio-visual stream
c. the categorized psycho-social states,
d. at least a part of the contents of the first and/or second audio-visual stream converted to text,
e. received user input data
f. additional physiological data obtained from additional sensors,
wherein optionally the method further comprising a step of post-processing (S9) data for a plurality of sessions in the database, said post-processing comprising at least one of
identification of reaction patterns in a session which leads to a non-favourable result based on previous session series, and
determine how mirroring patterns develop over time.
